# EUROPEAN PATENT APPLICATION

(11) **EP 2 745 860 A1**
(43) Date of publication of application: **25.06.2014**
(21) Application number: 12824176.7
(22) Date of filing: 10.08.2012
(51) Int. Cl.: A61M 1/14

(54) **BLOOD PURIFICATION DEVICE**

(30) Priority: 17.08.2011 JP 2011178214
(71) Applicant: Nikkiso Company Limited, Tokyo 150-6022 (JP)
(72) Inventor: FURUHASHI, Tomohiro, Makinohara-shi Shizuoka 421-0496 (JP); SUGIOKA, Akira, Makinohara-shi Shizuoka 421-0496 (JP); TOYODA, Masahiro, Makinohara-shi Shizuoka 421-0496 (JP)
(74) Representative: von Hirschhausen, Helge
(86) International application number: PCT/JP2012/070545
(87) International publication number: WO 2013/024825

(57) **Abstract**

[Object] To provide a blood purification apparatus which can reliably substitute blood during returning of blood and suppress a supply amount of a substitution solution.

[Solution] The blood purification apparatus includes a blood circuit that is formed to have an arterial blood circuit 1 and a venous blood circuit 2 and that can extracorporeally circulate blood of a patient from a tip of the arterial blood circuit 1 to a tip of the venous blood circuit 2; and a dialyzer 3 that is interposed between the arterial blood circuit 1 and the venous blood circuit 2 and that purifies the blood. The blood purification apparatus can substitute the blood inside the blood circuit during returning of the blood after treatment. The blood purification apparatus further includes an air circulation line that is connected to a predetermined portion in the blood circuit and that can circulate air; an air pump 18 that can supply the air into the blood circuit via the air circulation line; and a control device 19 that controls the air pump 18 to supply the air into the blood circuit during returning of the blood.

## Description

The present invention relates to a blood purification apparatus for extracorporeally circulating the blood of a patient to purify the blood in dialysis treatment using a dialyzer.

### Background Art

A hemodialysis apparatus as a blood purification apparatus is mainly configured to include a blood circuit consisting of an arterial blood circuit having an arterial puncture needle and a venous blood circuit having a venous puncture needle; a dialyzer which is interposed between the arterial blood circuit and the venous blood circuit and purifies blood flowing in the blood circuit; a blood pump which is arranged in the arterial blood circuit; an artery side air trap chamber and a vein side air trap chamber which are respectively arranged in the arterial blood circuit and the venous blood circuit; and a dialysis device which can supply a dialysate to the dialyzer.

In addition, a containing device (so-called saline bag) which contains a physiological saline solution is connected to between a tip in the arterial blood circuit and the blood pump via a physiological saline solution supplying line, thereby enabling cleaning/priming before dialysis treatment, substitution during the dialysis treatment and returning of blood after the dialysis treatment. For example, during returning of the blood, the physiological saline solution inside the containing device is supplied into the blood circuit via the physiological saline solution supplying line, and the blood inside the blood circuit is substituted with the physiological saline solution, thereby enabling the blood to reflow into a body of a patient. For example, PTL 1 discloses returning blood work of a dialysis apparatus in the related art.

### Citation List

### Patent Literature

PTL 1: Japanese Unexamined Patent Application Publication No. 2004-222884

### Summary of Invention

### Technical Problem

However, the above-described blood purification apparatus in the related art has the following problem.

During returning of the blood after the treatment, returning of the blood is supposed to be completed in theory if a substitution solution (physiological saline solution) is supplied to the blood circuit by an amount equivalent to an extracorporeally circulating amount of blood in the blood circuit (total capacity of the blood circuit and the air trap chambers). However, in practice, the substitution solution having the extracorporeally circulating amount or more is needed. This is due to a fact that if there is a stay portion of flowing in a flow route of the blood such as the blood circuit and the air trap chambers, the blood (particularly, blood corpuscle constituent in blood) is caused to stay in the stay portion and smooth substitution is hindered.

When returning of the blood by supplying a large amount of the substitution solution to the blood circuit in order to more reliably substitute the blood with the substitution solution, treatment costs are increased due to an increased amount of the substitution solution to be used and working hours for returning the blood are prolonged, thereby causing a patient to have heavy burden. In addition, when returning of the blood by supplying a large amount of the substitution solution to the blood circuit, there is a problem in that a large amount of the substitution solution is introduced into the body of the patient together with the blood.

The present invention is made in view of such circumstances, and aims to provide the blood purification apparatus which can reliably substitute the blood during returning of the blood and can suppress a supply amount of the substitution solution.

### Solution to Problem

According to the invention described in Claim 1, there is provided a blood purification apparatus including a blood circuit that consists of an arterial blood circuit and a venous blood circuit and that can extracorporeally circulate blood of a patient from a tip of the arterial blood circuit to a tip of the venous blood circuit; and a blood purification device that is interposed between the arterial blood circuit and the venous blood circuit in the blood circuit and that purifies the blood flowing in the blood circuit. The blood purification apparatus can return blood by substituting the blood inside the blood circuit after treatment. The blood purification apparatus further includes an air circulation line that is connected to a predetermined portion in the blood circuit and that can circulate air. During returning of the blood, the air can be supplied into the blood circuit via the air circulation line.

According to the invention described in Claim 2, the blood purification apparatus described in Claim 1 further includes an air supplying device that can supply the air into the blood circuit via the air circulation line; and a control device that controls the air supplying device to supply the air into the blood circuit during returning of the blood.

According to the invention described in Claim 3, the blood purification apparatus described in Claim 1 or 2 further includes an air trap chamber that is connected to the arterial blood circuit or the venous blood circuit. The air can be supplied by connecting the air circulation line to the air trap chamber.

According to the invention described in Claim 4, the blood purification apparatus described in Claim 3 further includes a liquid surface detection sensor that can detect a liquid surface of the air trap chamber. Under a condition that the liquid surface sensor detects the liquid surface, the air circulation line stops supplying the air.

According to the invention described in Claim 5, in the blood purification apparatus described in Claim 3 or 4, the air supplying device can supply the air to or discharge the air from the air trap chamber and can adjust the liquid surface inside the air trap chamber.

According to the invention described in Claim 6, in the blood purification apparatus described in any one of Claims 1 to 5, an air bubble detection sensor which can detect an air bubble inside a flow route of a tip portion of the arterial blood circuit and the venous blood circuit and a valve device which can open and close the flow route of the tip portion are arranged in the tip portion. During returning of the blood, under a condition that the air bubble detection sensor detects the air bubble, the valve device is in a closed state and the air circulation line stops supplying the air.

According to the invention described in Claim 7, the blood purification apparatus according to any one of Claims 1 to 6 further includes a containing device that contains a predetermined amount of a physiological saline solution as a substitution solution; and substitution solution supplying device that has a physiological saline solution supplying line which can supply the physiological saline solution inside the containing device into the blood circuit by connecting the containing device and a predetermined portion of the blood circuit to each other, and that can supply the substitution solution into the blood circuit.

According to the invention described in Claim 8, in the blood purification apparatus according to any one of Claims 1 to 6, the blood purification device can back-filtrate a dialysate as the substitution solution and supply the filtered dialysate into the blood circuit.

### Advantageous Effects of Invention

According to the invention described in Claim 1, the air circulation line supplies the air into the blood circuit during returning of the blood. Therefore, it is possible to reduce the amount of the blood to be substituted with the substitution solution by substituting the blood with the supplied air. Thus, it is possible to reliably substitute the blood during returning of the blood, and it is possible to suppress the supply amount of the substitution solution.

According to the invention described in Claim 2, the blood purification apparatus includes the air supplying device that can supply the air into the blood circuit via the air circulation line, and the control device that controls the air supplying device to supply the air into the blood circuit during returning of the blood. Therefore, it is possible to more reliably and accurately supply the air into the blood circuit during returning of the blood.

According to the invention described in Claim 3, the blood purification apparatus includes the air trap chamber that is connected to the arterial blood circuit or the venous blood circuit, and the air can be supplied by connecting the air circulation line to the air trap chamber. Therefore, it is possible to substitute the blood inside the air trap chamber which has a relatively large capacity and is likely to form a stay portion, with the air. Thus, it is possible to more reliably substitute the blood with the substitution solution during returning of the blood, and it is possible to suppress the supply amount of the substitution solution.

According to the invention described in Claim 4, the blood purification apparatus includes the liquid surface detection sensor that can detect the liquid surface of the air trap chamber, and under the condition that the liquid surface sensor detects the liquid surface, the air circulation line stops supplying the air. Therefore, it is possible to suppress the supply amount of the substitution solution by at least a change amount of the liquid surface.

According to the invention described in Claim 5, the air supplying device can supply the air to or discharge the air from the air trap chamber and can adjust the liquid surface inside the air trap chamber. Therefore, in addition to a function of supplying the air during returning of the blood after the treatment, it is possible to provide a function of adjusting the liquid surface in the air trap chamber before the treatment or during the treatment.

According to the invention described in Claim 6, the air bubble detection sensor which can detect an air bubble inside the flow route of the tip portion of the arterial blood circuit and the venous blood circuit and the valve device which can open and close the flow route of the tip portion are arranged in the tip portion. During returning of the blood, under the condition that the air bubble detection sensor detects the air bubble, the valve device is in the closed state and the air circulation line stops supplying the air. Therefore, it is not necessary to perform the substitution by supplying the substitution solution into the blood circuit during returning of the blood.

According to the invention described in Claim 7, the blood purification apparatus includes the containing device that contains the predetermined amount of the physiological saline solution as the substitution solution; and the substitution solution supplying device that has the physiological saline solution supplying line which can supply the physiological saline solution inside the containing device into the blood circuit by connecting the containing device and the predetermined portion of the blood circuit to each other, and that can supply the substitution solution into the blood circuit. Therefore, it is possible to perform returning of the blood by adopting a general returning blood method of using the physiological saline solution as the substitution solution.

According to the invention described in Claim 8, the blood purification device can back-filtrate the dialysate as the substitution solution and supply the filtered dialysate into the blood circuit. Therefore, it is possible to eliminate a need for a dedicated pipe for supplying the substitution solution, and it is possible to simplify the pipe.

### Brief Description of Drawings

[Fig. 1] Fig. 1 is a schematic view illustrating a blood purification apparatus (state when starting returning of blood after treatment) according to a first embodiment of the present invention.
[Fig. 2] Fig. 2 is a schematic view illustrating the blood purification apparatus (state where air and a substitution solution are supplied during returning of the blood).
[Fig. 3] Fig. 3 is a schematic view illustrating the blood purification apparatus (state where the substitution solution is supplied during returning of the blood).
[Fig. 4] Fig. 4 is a schematic view illustrating a blood purification apparatus (state where air and a substitution solution are supplied during returning of blood) according to another embodiment of the present invention.
[Fig. 5] Fig. 5 is a schematic view illustrating a blood purification apparatus (state where returning of blood is performed using back-filtration) according to a second embodiment of the present invention.
[Fig. 6] Fig. 6 is a schematic view illustrating an artery side air trap chamber in another embodiment which is applied to the blood purification apparatus.
[Fig. 7] Fig. 7 is a schematic view illustrating a blood purification apparatus (state where air and a substitution solution are supplied during returning of blood) according to a third embodiment of the present invention.

### Description of Embodiments

Hereinafter, an embodiment of the present invention will be described in detail with reference to the drawings.

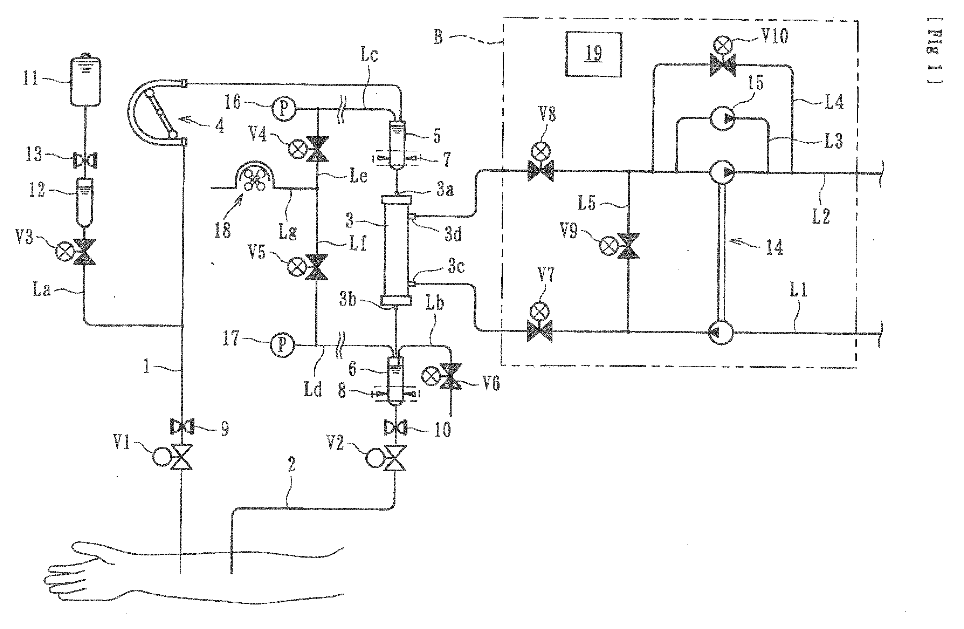

A blood purification apparatus according to a first embodiment consists of a hemodialysis apparatus for performing hemodialysis treatment, and as illustrated in Fig. 1, is mainly configured to include a blood circuit that consists of an arterial blood circuit 1 and a venous blood circuit 2; a dialyzer 3 (blood purification device) that is interposed between the arterial blood circuit 1 and the venous blood circuit 2 and purifies blood flowing in the blood circuit; a peristaltic blood pump 4 that is arranged in the arterial blood circuit 1; an artery side air trap chamber 5 and a vein side air trap chamber 6 which are respectively connected to an intermediate portion of the arterial blood circuit 1 and the venous blood circuit 2; a dialysis device B which can supply a dialysate to the dialyzer 3; and a control device 19 that is arranged inside the dialysis device B.

In the arterial blood circuit 1, an arterial puncture needle is connected to a tip thereof, and the peristaltic blood pump 4 and the artery side air trap chamber 5 for removing air bubbles are arranged in an intermediate portion thereof. In contrast, in the venous blood circuit 2, a venous puncture needle is connected to a tip thereof, and the vein side air trap chamber 6 for removing the air bubble is connected to an intermediate portion thereof. In addition, a physiological saline solution supplying line La extending from a containing device 11 is connected to a predetermined portion (portion between an air bubble detection sensor 9 and the blood pump 4) of the arterial blood circuit 1.

The containing device 11 is a so-called "saline bag" and contains a predetermined amount of a physiological saline solution as a substitution solution. The physiological saline solution supplying line La can supply the physiological saline solution inside the containing device 11 into the blood circuit by connecting the containing device 11 and a predetermined portion in the blood circuit (portion between the air bubble detection sensor 9 and the blood pump 4) to each other. The containing device 11 and the physiological saline solution supplying line La configure a "substitution solution supplying device" which can supply the substitution solution into the blood circuit. An electromagnetic valve V3 which can arbitrarily open and close a flow route thereof is arranged in the physiological saline solution supplying line La according to the present embodiment, to which an air bubble detection sensor 13 (liquid shortage sensor) is connected in order to detect a liquid level in an air trap chamber 12 and the containing device 11.

The artery side air trap chamber 5 and the vein side air trap chamber 6 can remove the air bubble by capturing the air bubble in the blood inside the blood circuit, has a filtration net (not illustrated) arranged thereinside and can capture a thrombus during returning of blood, for example. Monitor tubes Lc and Ld respectively extend from each upper portion (air layer side) of the artery side air trap chamber 5 and the vein side air trap chamber 6, and tips thereof are respectively connected to pressure sensors 16 and 17. The monitor tubes Lc and Ld and the pressure sensors 16 and 17 can respectively measure a liquid pressure inside the artery side air trap chamber 5 (dialyzer inlet pressure) and a liquid pressure inside the vein side air trap chamber 6 (venous pressure).

Furthermore, the artery side air trap chamber 5 and the vein side air trap chamber 6 have liquid surface detection sensors 7 and 8 which can detect each liquid surface. The liquid surface detection sensors 7 and 8 are configured to have a sensor which can detect that the liquid surface is lowered down to a lower portion of the artery side air trap chamber 5 or the vein side air trap chamber 6. In addition, an overflow line Lb extends from an upper portion of the vein side air trap chamber 6, and an electromagnetic valve V6 which can arbitrarily open and close the overflow line Lb is arranged in an intermediate portion thereof.

In contrast, in a tip portion of the arterial blood circuit 1 (vicinity of the arterial puncture needle), the air bubble detection sensor 9 which can detect the air bubble inside a flow route of the tip portion and an electromagnetic valve V1 as a valve device which can open and close the flow route of the tip portion (vicinity of an upstream side of the air bubble detection sensor 9) are arranged. In a tip portion of the venous blood circuit 2 (vicinity of the venous puncture needle), an air bubble detection sensor 10 which can detect the air bubble inside a flow route of the tip portion and an electromagnetic valve V2 as a valve device which can open and close the flow route of the tip portion (vicinity of an upstream side of the air bubble detection sensor 10) are arranged. The air bubble detection sensors 9 and 10 and the electromagnetic valves V1 and V2 are generally fixed to the dialysis device B, but may be a separate installation type (for example, a clip style) so as to be respectively installed at any position in the arterial blood circuit 1 and the venous blood circuit 2.

Then, if the blood pump 4 is rotated in a state where the arterial puncture needle and the venous puncture needle are punctured a patient, the blood of the patient reaches the dialyzer 3 through the arterial blood circuit 1 and then the blood is purified by the dialyzer 3. Then, the blood reflows into a body of the patient through the venous blood circuit 2 while the air bubble is removed in the vein side air trap chamber 6. That is, the blood of the patient is purified by the dialyzer 3 while the blood is extracorporeally circulated from the tip of the arterial blood circuit 1 to the tip of the venous blood circuit 2 in the blood circuit.

In the dialyzer 3, a housing unit thereof has a blood inlet port 3a, a blood outlet port 3b, a dialysate inlet port 3c and a dialysate outlet port 3d. Among them, the arterial blood circuit 1 is connected to the blood inlet port 3a and the venous blood circuit 2 is connected to the blood outlet port 3b respectively. In addition, the dialysate inlet port 3c and the dialysate outlet port 3d are respectively connected to a dialysate introduction line L1 and a dialysate discharge line L2, each of which extends from the dialysis device B.

The dialyzer 3 accommodates a plurality of hollow fibers inside thereof. An interior of the hollow fibers serves as the flow route of the blood and a space between an outer peripheral surface of the hollow fibers and an inner peripheral surface of the housing unit serves as the flow route of the dialysate. The hollow fiber has a hollow fiber membrane by forming multiple minute holes (pores) penetrating the outer peripheral surface and the inner peripheral surface thereof. In this configuration, impurities in the blood can be transmitted into the dialysate via the membrane.

The dialysis device B has a duplex pump 14 which is arranged across the dialysate introduction line L1 and the dialysate discharge line L2, an ultrafiltration pump 15 for removing water from the patient's blood flowing in the dialyzer 3, and a control device 19. One end of the dialyzer introduction line L1 is connected to the dialyzer 3 (dialysate inlet port 3c) and the other end is connected to a dialysate supplying apparatus (not illustrated) which produces the dialysate having predetermined concentration. In addition, one end of the dialysate discharge line L2 is connected to the dialyzer 3 (dialysate outlet port 3d) and the other end is connected to a liquid discharge device (not illustrated). Rotation of the duplex pump 14 causes the dialysate supplied from the dialysate supplying apparatus to reach the dialyzer 3 through the dialysate introduction line L1 and to be sent to the liquid discharge device through the dialysate discharge line L2.

Further, a bypass flow route L3 which bypasses a liquid discharge side of the duplex pump 14 is extended to the dialysate discharge line L2, and the ultrafiltration pump 15 is arranged in an intermediate portion of the bypass flow route L3. Furthermore, a bypass flow route L4 which bypasses the duplex pump 14 and the bypass flow route L3 is extended to the dialysate discharge line L2, and an electromagnetic valve V10 which can arbitrarily open and close the flow route is arranged in the intermediate portion of the bypass flow route L4. A reference numeral L5 in the drawing represents a bypass flow route which connects the dialysate introduction line L1 and the dialysate discharge line L2 to each other and bypasses the dialyzer 3. An electromagnetic valve V9 which can arbitrarily open and close the flow route is arranged in the bypass flow route L5.

Here, the present embodiment includes air circulation lines (Le, Lf and Lg) which are connected to a predetermined portion in the blood circuit and through which the air can flow, an air pump 18 (air supplying device) which can supply the air into the blood circuit via the air circulation lines (Le, Lf and Lg), and a control device 19 which controls the air pump 18 to supply the air into the blood circuit during returning of blood. For convenience of layout in the drawing, pressure sensors 16 and 17 and the air pump 18 (to be described later) as the air supplying device are illustrated as if they are located in a position different from that of the dialysis device B, but are actually arranged in the dialysis device B.

The air circulation line consists of a flexible tube which can circulate the air, and is mainly configured to have the flow route Le whose tip is connected to the intermediate portion of the monitor tube Lc, the flow route Lf whose tip is connected to the intermediate portion of the monitor tube Ld and the flow route Lg whose tips are respectively connected to base ends of the flow routes Le and Lf and whose base end is exposed to the air. That is, the tip of the flow route Lg is divided into the flow routes Le and Lf, and the respective tips of the flow routes Le and Lf are connected to the intermediate portions of the monitor tubes Lc and Ld.

In this manner, the flow routes Lg and Le configuring the air circulation line are connected to an upper portion of the artery side air trap chamber 5 via the monitor tube Lc, and the flow routes Lg and Lf similarly configuring the air circulation line are connected to an upper portion of the vein side air trap chamber 6 via the monitor tube Ld. In addition, an electromagnetic valve V4 which can arbitrarily open and close the flow route thereof is arranged in the intermediate portion of the flow route Le, and an electromagnetic valve V5 which can arbitrarily open and close the flow route thereof is arranged in the intermediate portion of the flow route Lf.

The air pump 18 consists of a peristaltic pump, is arranged in the flow route Lg configuring the air circulation line and can be in a normal rotation (rotation to the right in the drawing) and in a reverse rotation (rotation to the left in the drawing). If the air pump 18 is in the normal rotation, the air is sucked by the base end of the flow route Lg and the air can be supplied into the artery side air trap chamber 5 or the vein side air trap chamber 6. In addition, if the air pump 18 is in the reverse rotation, the air is sucked by the air layer side of the artery side air trap chamber 5 or the vein side air trap chamber 6 and the air can be discharged by the base end of the flow route Lg.

In this manner, when the air pump 18 performs the normal rotation, the liquid surface of the artery side air trap chamber 5 or the vein side air trap chamber 6 can be lowered, and when the air pump 18 is in the reverse rotation, the liquid surface of the artery side air trap chamber 5 or the vein side air trap chamber 6 can be raised. Since the air pump 18 according to the present embodiment can be in the normal rotation and in the reverse rotation, the air can be supplied to (during the normal rotation) or discharged from (during the reverse rotation) the artery side air trap chamber 5 or the vein side air trap chamber 6. In this configuration, it is possible to adjust the liquid surface inside the artery side air trap chamber 5 and the vein side air trap chamber 6.

The control device 19 consists of a microcomputer arranged in the dialysis device B for example, which is electrically connected to an actuator configuring this blood purification apparatus (blood pump 4, air pump 18, duplex pump 14, ultrafiltration pump 15 and the like), electromagnetic valves V1 to V10 and various sensors (liquid surface detection sensors 7 and 8, air bubble detection sensors 9 and 10, pressure sensors 16 and 17 and the like). The control device 19 can automatically control a priming operation before treatment, various treatment operations during the treatment and returning of blood after the treatment.

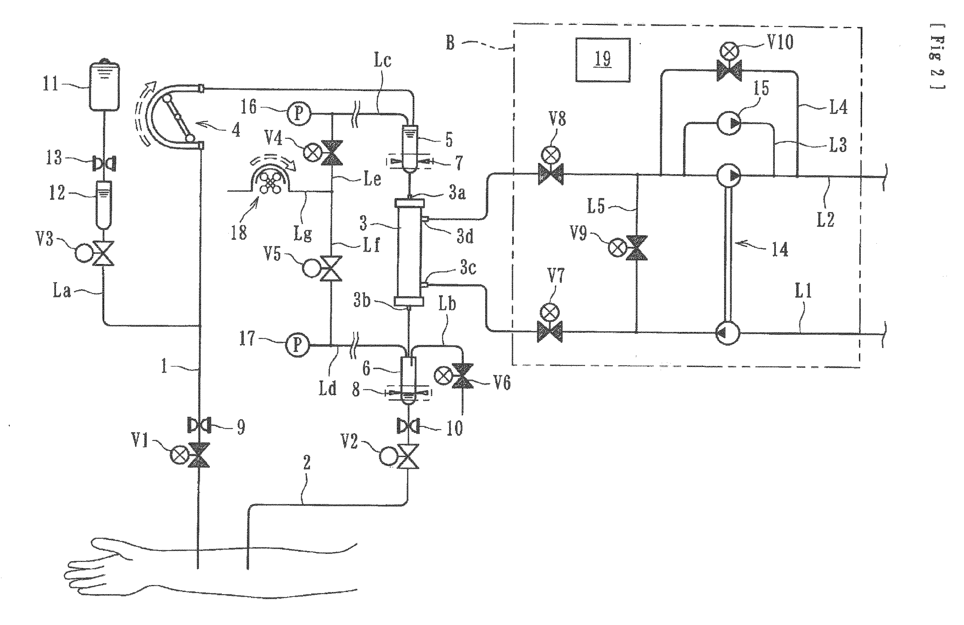

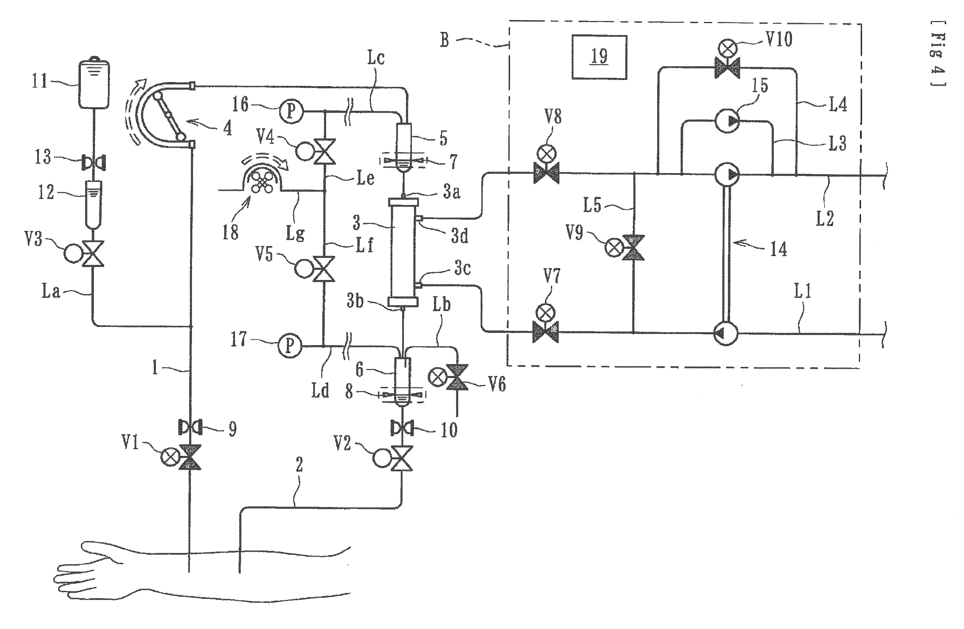

Thus, in addition to a series of controls relating to the treatment, the control device 19 according to the present embodiment is configured to be capable of supplying the air into the blood circuit via the artery side air trap chamber 5 or the vein side air trap chamber 6 by controlling the rotation of the air pump 18 at a predetermined time during returning of the blood. For example, during returning of the blood, the control device 19 leaves the electromagnetic valve V4 in a closed state and the electromagnetic valve V5 in an opened state, and causes the air pump 18 to be in the normal rotation (refer to Fig. 2) so as to enable the air to be supplied to the vein side air trap chamber 6 only. The control device 19 leaves the electromagnetic valves V4 and V5 in the opened state, and causes the air pump 18 to be in the normal rotation (refer to Fig. 4) so as to enable the air to be supplied to both of the artery side air trap chamber 5 and the vein side air trap chamber 6.

Next, a control method during returning of the blood in the blood purification apparatus according to the present embodiment will be described.

As illustrated in Fig. 1, after blood purification treatment is completed, the blood of the patient remains inside the blood circuit (arterial blood circuit 1 and venous blood circuit 2), the artery side air trap chamber 5 and the vein side air trap chamber 6, thereby requiring returning of the blood for causing the blood to reflow into the body of the patient. A process may be automatically transited to a returning blood process after the treatment is completed or may be manually transited to the returning blood process after the treatment is completed. In addition, in the present embodiment, any one of the electromagnetic valves V1 and V2 is in the opened state when starting returning of the blood, but the electromagnetic valves V1 and V2 may be in the closed state.

As illustrated in Fig. 2, under a condition that returning of the blood is started, the control device 19 leaves the electromagnetic valve V1 in the closed state, leaves the electromagnetic valves V2 and V3 in the opened state, leaves the electromagnetic valve V4 in the closed state and leaves the electromagnetic valve V5 in the opened state. At this time, the electromagnetic valves V6 to V10 are left in the closed state and the duplex pump 14 and the ultrafiltration pump 15 are left in a stopped state. However, the duplex pump 14 may be left in a rotated state and in this case, the electromagnetic valve V9 is left in the opened state. Then, the control device 19 controls the blood pump 4 to be in the normal rotation so as to supply the physiological saline solution (substitution solution) inside the containing device 11 to the blood circuit via the physiological saline solution supplying line La. The control device 19 controls the air pump 18 to be in the normal rotation so as to supply the air to the vein side air trap chamber 6 via the flow routes Lg and Lf and the monitor tube Ld which configure the air circulation line.

In this manner, the blood inside the vein side air trap chamber 6 is substituted with the air and the liquid surface is lowered. The blood inside the venous blood circuit 2, the blood in the further downstream side (blood pump 4 side) from a connection portion to the physiological saline solution supplying line La in the arterial blood circuit 1 and the blood inside the artery side air trap chamber 5 are substituted with the physiological saline solution, thereby allowing returning of the blood into the body of the patient. That is, during returning of the blood, it is possible to reduce the blood inside the vein side air trap chamber 6 by supplying the air to the vein side air trap chamber 6 so as to lower the liquid surface. Thus, it is possible to reduce the supply amount (use amount) of the physiological saline solution (substitution solution) to be substituted, by at least the reduced amount of the blood.

Thereafter, if the liquid surface detection sensor 8 detects the liquid surface (that is, if a state is detected where almost everything inside the vein side air trap chamber 6 is substituted with the air), the rotation of the air pump 18 is stopped and the supply of the air is stopped. Then, under a condition that the blood pump 4 supplies a predetermined amount of the physiological saline solution or a blood discrimination device (not illustrated) disposed in the tip of the venous blood circuit 2 detects that the blood is substituted with the physiological saline solution, the rotation of the blood pump 4 is stopped to leave the electromagnetic valve V2 in the closed state. When the rotation of the air pump 18 is stopped, both of the electromagnetic valves V4 and V5 may be controlled to be left in the closed state.

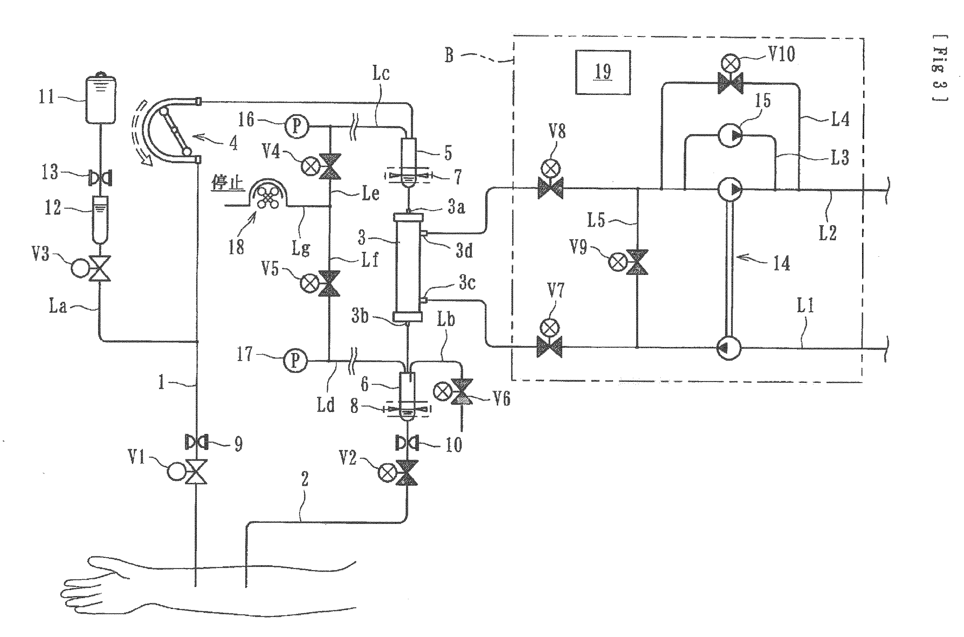

In this way, returning the blood of the venous blood circuit 2 side is completed. Subsequently, returning the blood of the arterial blood circuit 1 side is started. If returning the blood of the arterial blood circuit 1 side is started, the control device 19 controls the electromagnetic valves V1 and V2 to be in the closed state, controls the electromagnetic valve V3 to be in the opened state and controls the blood pump 4 to be in the normal rotation. This enables the vein side air trap chamber 6 to store the physiological saline solution by an empty capacity thereof (a capacity of the air layer after the liquid level is lowered). Then, as illustrated in Fig. 3, the control device 19 controls the blood pump 4 to be in the reverse rotation and causes the physiological saline solution stored in the vein side air trap chamber 6 to flow in the arterial blood circuit 1 so as to perform returning of the blood. At this time, the electromagnetic valve V1 is left in the opened state, and the electromagnetic valve V2 is left in the closed state. While maintaining the closed state of the electromagnetic valve V4, the electromagnetic valve V5 is left in the closed state. In this way, returning of the blood controlled by the control device 19 is completed.

Instead of the above-described manner, returning the blood of the arterial blood circuit 1 side can be performed in the following manner. That is, if returning the blood of the arterial blood circuit 1 side is started, the control device 19 controls the electromagnetic valve V2 to be in the closed state and controls the electromagnetic valves V1 and V3 to be in the opened state so as to maintain a stopped state of the blood pump 4. In this manner, self-weight of the physiological saline solution causes the physiological saline solution inside the containing device 11 to flow into the arterial blood circuit 1 via the physiological saline solution supplying line La so as to be substituted with the blood.

Thus, an operation to lower the liquid surface of the vein side air trap chamber 6 which is caused by the normal rotation of the air pump 18 is preferably completed at the latest until the physiological saline solution supplied by the rotation of the blood pump 4 reaches the vein side air trap chamber 6. In addition, in the present embodiment, the operation to lower the liquid surface of the vein side air trap chamber 6 which is caused by the normal rotation of the air pump 18 is performed until the liquid surface detection sensor 8 detects the liquid surfaces. However, instead of this manner, the operation may be set to be performed until the number of rotations or the rotation time of the air pump 18 reaches a predetermined value.

Here, the above-described embodiment is configured such that rotation of the air pump 18 enables the air to be supplied into the blood circuit via the air circulation lines (Le, Lf and Lg). However, instead of the air pump 18, an electromagnetic valve may be arranged. In this case, for example, if the electromagnetic valve arranged instead of the air pump 18 and the electromagnetic valve V4 or the electromagnetic valve V5 are left in the opened state and the electromagnetic valves V8 and V10 inside the dialysis device B are left in the opened state, the self-weight of the dialysate causes the dialysate inside the dialyzer 3 to flow to the dialysate discharge line L2 side. In this manner, it is possible to introduce the air from the tip of the air circulation line Lg and to supply the air into the blood circuit. That is, if the electromagnetic valves V8 and V10 are left in the opened state, the pipe of the dialysate inside the blood circuit and the dialysis device B is in an exposed state to the air. Then, potential energy based on a difference in a height between the artery side air trap chamber 5 or the vein side air trap chamber 6 and the electromagnetic valve V10 causes moisture of the blood in the blood circuit to be filtered and thus the blood is caused to move to the dialysate discharge line L2 side. Therefore, it is possible to introduce the air from the tip of the air circulation line Lg and to supply the air to the artery side air trap chamber 5 or the vein side air trap chamber 6.

In the present embodiment, when starting returning of the blood, the air pump 18 is rotated and the blood pump 4 is in the normal rotation. However, instead of this manner, when starting returning of the blood, after the air pump 18 is rotated to supply the air into the vein side air trap chamber 6 and the blood inside the vein side air trap chamber 6 is substituted with the air (after the liquid surface detection sensor 8 detects the liquid surface), the blood pump 4 may be in the normal rotation or in the reverse rotation.

Furthermore, in the above-described embodiment, during returning of the blood, the air is supplied to the vein side air trap chamber 6 only. However, instead of this manner, as illustrated in Fig. 4, during returning of the blood, the control device 19 may control both of the electromagnetic valves V4 and V5 to be in the opened state and may control the air pump 18 to be in the normal rotation. In this case, it is possible to supply the air to both of the artery side air trap chamber 5 and the vein side air trap chamber 6 by rotation of the air pump 18 until the liquid surface detection sensors 7 and 8 respectively detect the liquid surface.

According to the first embodiment as described above, during returning of the blood, the air pump 18 (air supplying device) is controlled to supply the air into the blood circuit. Therefore, it is possible to reduce the amount of the blood to be substituted with the physiological saline solution (substitution solution) by substituting the blood with the supplied air. Consequently, it is possible to reliably substitute the blood with the physiological saline solution during returning of the blood and it is possible to suppress the supply amount of the physiological saline solution.

In addition, there are provided the artery side air trap chamber 5 and the vein side air trap chamber 6 which are respectively connected to the arterial blood circuit 1 and the venous blood circuit 2. The air circulation line is connected to the artery side air trap chamber 5 and the vein side air trap chamber 6, and the air pump 18 can supply the air to the air circulation line. Therefore, the blood inside the artery side air trap chamber 5 and the vein side air trap chamber 6 which have a relatively large capacity and are likely to form a stay portion can be substituted with the air. Consequently, it is possible to more reliably substitute the blood with the physiological saline solution during returning of the blood and it is possible to suppress the supply amount of the physiological saline solution.

In addition, according to the present embodiment, there is provided the liquid surface detection sensor 8 which can detect the liquid surface of the vein side air trap chamber 6. Under a condition that the liquid surface detection sensor 8 detects the liquid surface, the control device 19 stops the air pump 18 so as not to supply the air. Therefore, it is possible to suppress the supply amount of the physiological saline solution by the change amount of the liquid surface. According to another embodiment as illustrated in Fig. 4, under a condition that the liquid surface detection sensors 7 and 8 detect the liquid surface, the control device 19 stops the air pump 18 so as not to supply the air. Consequently, it is possible to suppress the supply amount of the physiological saline solution by at least a total changed amount of the liquid surface of the artery side air trap chamber 5 and the vein side air trap chamber 6.

Further, the air pump 18 can supply the air to or discharge the air from the artery side air trap chamber 5 and the vein side air trap chamber 6. Thus, the liquid surface inside the artery side air trap chamber 5 and the vein side air trap chamber 6 can be adjusted. Therefore, in addition to a function of supplying the air during returning of the blood after the treatment, it is possible to provide a function of adjusting the liquid surface in the artery side air trap chamber 5 and the vein side air trap chamber 6 before the treatment or during the treatment.

Furthermore, there are provided the containing device 11 which contains the predetermined amount of physiological saline solution as the substitution solution, and the substitution solution supplying device that has the physiological saline solution supplying line La which connects the containing device 11 and the predetermined portion of the blood circuit to each other and can supply the physiological saline solution inside the containing device 11 into the blood circuit, and that can supply the substitution solution into the blood circuit. Therefore, it is possible to perform returning of the blood by adopting the general returning blood method of using the physiological saline solution as the substitution solution.

Next, a blood purification apparatus according to a second embodiment of the present invention will be described.

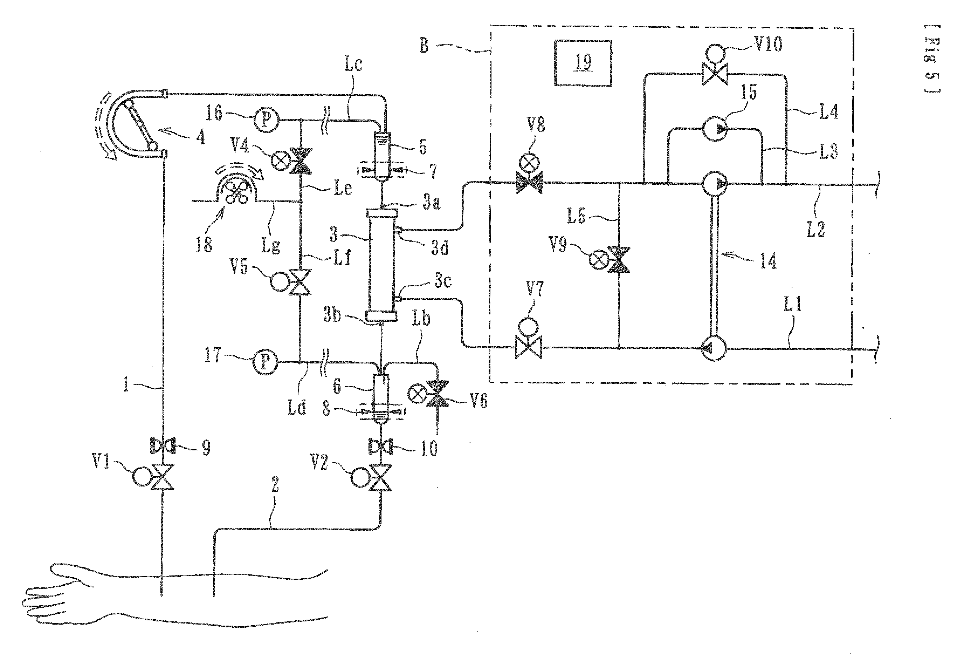

Similar to the first embodiment, the blood purification apparatus according to the present embodiment consists of a hemodialysis apparatus for performing hemodialysis treatment, and as illustrated in Fig. 5, is mainly configured to include a blood circuit that consists of the arterial blood circuit 1 and the venous blood circuit 2; the dialyzer 3 (blood purification device) that is interposed between the arterial blood circuit 1 and the venous blood circuit 2 and purifies the blood flowing in the blood circuit; the peristaltic blood pump 4 that is arranged in the arterial blood circuit 1; the artery side air trap chamber 5 and the vein side air trap chamber 6 which are respectively connected to the intermediate portion of the arterial blood circuit 1 and the venous blood circuit 2; the dialysis device B which can supply the dialysate to the dialyzer 3; and the control device 19 that is arranged inside the dialysis device B. The same reference numerals are given to configuring components the same as those of the first embodiment, and description thereof will be omitted.

The dialyzer 3 (blood purification device) according to the present embodiment can perform back-filtration on the dialysate as the substitution solution and supply the filtered dialysate into the blood circuit. Specifically, as illustrated in Fig. 5, during returning of the blood, the control device 19 controls the electromagnetic valves V1 and V2 to be in the opened state, controls the electromagnetic valves V5, V7 and V10 to be in the opened state and controls the electromagnetic valves V4, V8 and V9 to be in the closed state.

In such a state, the control device 19 causes the air pump 18 to be rotated, the duplex pump 14 and the blood pump 4 (blood pump 4 is in the reverse rotation). At this time, the blood pump 4 is set to be in the reverse rotation at a speed slower than that of the duplex pump 14. Thus, the normal rotation of the air pump 18 allows the air to be supplied to the vein side air trap chamber 6, and the rotation of the duplex pump 14 enables the dialysate to be supplied by pumping to the dialysate flow route of the dialyzer 3 via the dialysate introduction line L1 so as to perform the back-filtration in the blood circuit side. Since the blood pump 4 is in the reverse rotation at a speed slower than that of the duplex pump 14, the dialysate subjected to the back-filtration flows in both of the arterial blood circuit 1 and the venous blood circuit 2 and is substituted with the blood in the respective blood circuits.

Then, under a condition that the liquid surface detection sensor 8 detects the liquid surface, the rotation of the air pump 18 is stopped and the blood pump 4 supplies the predetermined amount of physiological saline solution, or under a condition that the blood discrimination device (not illustrated) disposed at the tip portion of the arterial blood circuit 1 and the venous blood circuit 2 detects that the blood is substituted with the dialysate, the rotation of the blood pump 4 and the duplex pump 14 is stopped to leave the electromagnetic valves V1 and V2 in the closed state. In this manner as described above, returning of the blood is completed.

Therefore, it is preferable that the air pump 18 be rotated at such a speed that the liquid surface is lowered until the dialysate reaches the vein side air trap chamber 6 at the latest. In addition, in the present embodiment, the operation to lower the liquid surface of the vein side air trap chamber 6 by using the normal rotation of the air pump 18 is performed until the liquid surface detection sensor 8 detects the liquid surface. However, instead of this manner, the operation may be set to be performed until the number of rotations or the rotation time of the air pump 18 reaches a predetermined value.

In the present embodiment, when starting returning of the blood, the air pump 18 is rotated, and the blood pump 4 and the duplex pump 14 are rotated. However, instead of this manner, when starting returning of the blood, after the air pump 18 is rotated to supply the air into the vein side air trap chamber 6 and the blood inside the vein side air trap chamber 6 is substituted with the air (after the liquid surface detection sensor 8 detects the liquid surface), the blood pump 4 and the duplex pump 14 may be rotated.

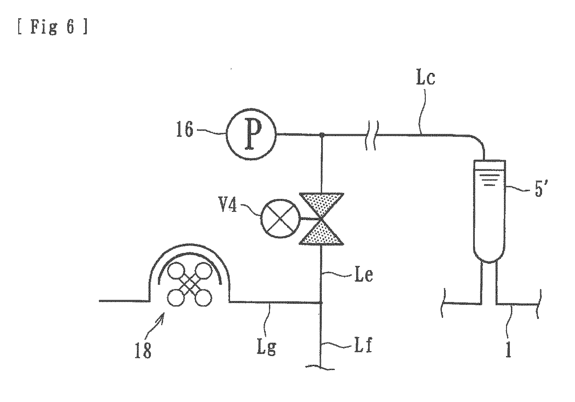

Furthermore, instead of the artery side air trap chamber 5 according to the present embodiment, as illustrated in Fig. 6, it is preferable to adopt an artery side air trap chamber 5' where an inlet port of the blood from the arterial blood circuit 1 and an outlet port of the blood to the arterial blood circuit 1 are formed in a lower portion of the air trap chamber. If such an artery side air trap chamber 5' is adopted, it is possible to more reliably and smoothly substitute the blood inside the arterial blood circuit 1 with the dialysate as the substitution solution and to perform returning of the blood.

According to the second embodiment as described above, similar to the first embodiment, during returning of the blood, the air pump 18 (air supplying device) is controlled to supply the air into the blood circuit. Therefore, it is possible to reduce the amount of the blood to be substituted with the dialysate (substitution solution) by substituting the blood with the supplied air. Consequently, it is possible to reliably substitute the blood with the dialysate during returning of the blood and it is possible to suppress the supply amount of the dialysate.

In addition, there are provided the artery side air trap chamber 5 and the vein side air trap chamber 6 which are respectively connected to the arterial blood circuit 1 and the venous blood circuit 2. The air circulation line is connected to the artery side air trap chamber 5 and the vein side air trap chamber 6, and the air pump 18 can supply the air to the air circulation line. Therefore, the blood inside the artery side air trap chamber 5 and the vein side air trap chamber 6 which have relatively large capacity and are likely to form the stay portion can be substituted with the air. Consequently, it is possible to more reliably substitute the blood with the dialysate during returning of the blood and it is possible to suppress the supply amount of the dialysate.

Further, the air pump 18 can supply the air to or can discharge the air from the artery side air trap chamber 5 and the vein side air trap chamber 6. Thus, the liquid surface inside the artery side air trap chamber 5 and the vein side air trap chamber 6 can be adjusted. Therefore, in addition to the function of supplying the air during returning of the blood after the treatment, it is possible to provide the function of adjusting the liquid surface in the artery side air trap chamber 5 and the vein side air trap chamber 6 before the treatment or during the treatment.

In addition, according to the present embodiment, there is provided the liquid surface detection sensor 8 which can detect the liquid surface of the vein side air trap chamber 6. Under the condition that the liquid surface detection sensor 8 detects the liquid surface, the control device 19 stops the air pump 18 so as not to supply the air. Therefore, it is possible to suppress the supply amount of the dialysate by the changed amount of the liquid surface. In particular, the dialyzer 3 (blood purification device) according to the present embodiment can perform the back-filtration on the dialysate as the substitution solution and supply the filtered dialysate into the blood circuit. Therefore, it is possible to eliminate a need for a dedicated pipe (for example, the physiological saline solution supplying line La as described in the first embodiment) for supplying the substitution solution, and it is possible to simplify the pipe. Furthermore, according to the present embodiment, it is not necessary to use the physiological saline solution as the substitution solution during returning of the blood. Therefore, it is not necessary to provide the substitution solution supplying device having the containing device 11 and the physiological saline solution supplying line La as described in the first embodiment.

In the present embodiment, the dialyzer 3 can perform the back-filtration on the dialysate as the substitution solution and supply the filtered dialysate into the blood circuit. However, instead of this manner, a flexible tube may be connected between the dialysate supplying line L1 and the blood circuit (for example, a portion between the blood pump 4 and the electromagnetic valve V1 in the arterial blood circuit 1) and the dialysate as the substitution solution may be supplied from the dialysate supplying line L1 to the blood circuit through the flexible tube. In addition, in this case, it is possible to substitute the blood with the dialysate by the duplex pump 14 (alternatively, a peristaltic dialysate substitution pump arranged in the flexible tube between the dialysate supplying line L1 and the blood circuit) to be rotated and by supplying the dialysate into the blood circuit.

Next, a blood purification apparatus according to a third embodiment of the present invention will be described.

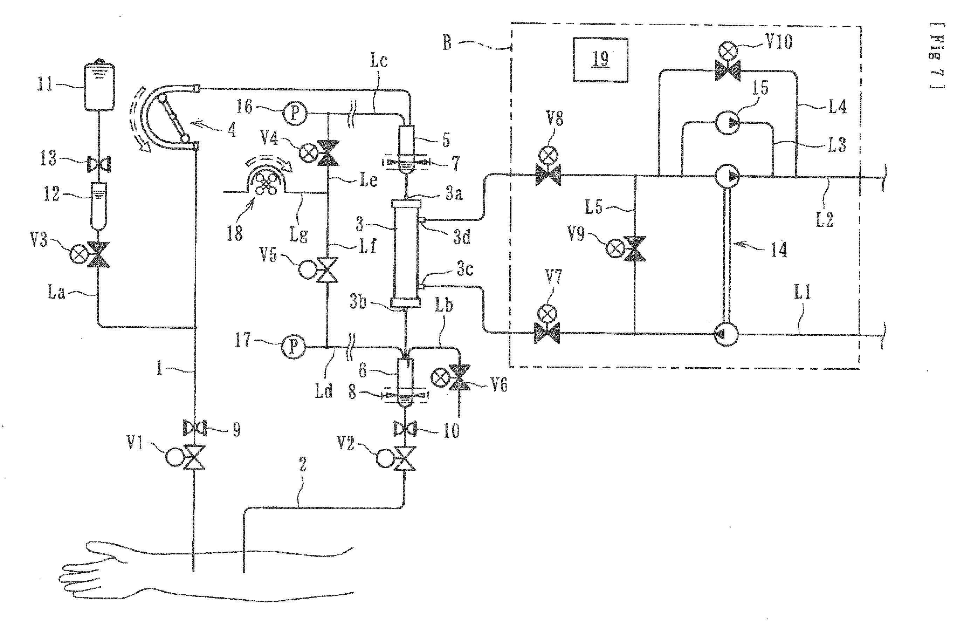

Similar to the first and second embodiments, the blood purification apparatus according to the present embodiment consists of a hemodialysis apparatus for performing hemodialysis treatment, and as illustrated in Fig. 7, is mainly configured to include a blood circuit that consists of the arterial blood circuit 1 and the venous blood circuit 2; the dialyzer 3 (blood purification device) that is interposed between the arterial blood circuit 1 and the venous blood circuit 2 and purifies the blood flowing in the blood circuit; the peristaltic blood pump 4 that is arranged in the arterial blood circuit 1; the artery side air trap chamber 5 and the vein side air trap chamber 6 which are respectively connected to the intermediate portion of the arterial blood circuit 1 and the venous blood circuit 2; the dialysis device B which can supply the dialysate to the dialyzer 3; and the control device 19 that is arranged inside the dialysis device B. The same reference numerals are given to configuring components the same as those of the first and second embodiments, and description thereof will be omitted.

Under a condition that the air bubble detection sensors 9 and 10 detect the air bubble, during returning of the blood, the control device 19 according to the present embodiment controls the electromagnetic valves V1 or V2 as the valve device to be in the closed state and controls the air pump 18 to stop supplying the air. That is, similar to the first and second embodiments, without using the substitution solution such as the physiological saline solution or the dialysate, the blood is configured to be substituted with the air supplied from the air pump 18.

More specifically, as illustrated in Fig. 7, during returning of the blood after the treatment is completed, the control device 19 controls the electromagnetic valves V1, V2 and V5 to be in the opened state and controls the electromagnetic valves V3, V4 and V6 to be in the closed state. Thereafter, the blood pump 4 is in the reverse rotation and the air pump 18 is in the normal rotation. At this time, the blood pump 4 is in the reverse rotation at a speed slower than that of the air pump 18. In this manner, the air supplied by the air pump 18 is set to be simultaneously supplied into both of the arterial blood circuit 1 and the venous blood circuit 2.

Then, under a condition that the air bubble detection sensor 9 detects the air bubble, the electromagnetic valve V1 is left in the closed state, and under a condition that the air bubble detection sensor 10 detects the air bubble, the electromagnetic valve V2 is left in the closed state. In this manner, returning of the blood can be simultaneously performed on the arterial blood circuit 1 and the venous blood circuit 2. Therefore, it is not necessary to substitute the blood by supplying the substitution solution (physiological saline solution or dialysate) to the blood circuit during returning of the blood.

Here, the above-described embodiment is configured such that rotation of the air pump 18 enables the air to be supplied into the blood circuit via the air circulation lines (Le, Lf and Lg). However, for example, instead of the air pump 18, an electromagnetic valve may be arranged. In this case, if the electromagnetic valve arranged instead of the air pump 18 and the electromagnetic valve V4 or the electromagnetic valve V5 are left in the opened state and the electromagnetic valves V8 and V10 inside the dialysis device B are left in the opened state, the self-weight of the dialysate causes the dialysate inside the dialyzer 3 to flow to the dialysate discharge line L2 side. In this manner, it is possible to introduce the air from the tip of the air circulation line Lg and to supply the air into the blood circuit. That is, if the electromagnetic valves V8 and V10 are left in the opened state, the pipe of the dialysate inside the blood circuit and the dialysis device B is in an exposed state to the air. Then, potential energy based on a difference in a height between the artery side air trap chamber 5 or the vein side air trap chamber 6 and the electromagnetic valve V10 causes moisture of the blood in the blood circuit to be filtered and thus the blood is caused to move to the dialysate discharge line L2. Therefore, it is possible to introduce the air from the tip of the air circulation line Lg and to supply the air to the artery side air trap chamber 5 or the vein side air trap chamber 6.

In the above-described embodiment, the electromagnetic valve V4 is left in the closed state and the electromagnetic valve V5 is left in the opened state, thereby supplying the air via the vein side air trap chamber 6. However, instead of this manner, a configuration may be made such that the air is supplied via the artery side air trap chamber 5 by leaving the electromagnetic valve V4 in the opened state and the electromagnetic valve V5 in the closed state. In addition, during returning of the blood, the rotation of the blood pump 4 and the air pump 18 may not be simultaneously started as compared to the present embodiment. For example, the air pump 18 may be first caused to be rotated and then the blood pump 4 may be in the reverse rotation. Furthermore, as described above, returning of the blood according to the method of the first embodiment for example may be performed to some extent before returning of the blood and then returning of the blood according to the present embodiment may be performed. Thus, in the present embodiment, it is preferable to configure to substitute the blood of more patients with the air by installing the air bubble detection sensors 9 and 10 and the electromagnetic valves V1 and V2 such that the arrangement positions thereof are close to the tip of the arterial blood circuit 1 or the tip of the venous blood circuit 2 (vicinity of the arterial puncture needle or the venous puncture needle).

According to the third embodiment as described above, similar to the first and second embodiments, during returning of the blood, the air pump 18 (air supplying device) is controlled to supply the air into the blood circuit. Therefore, it is possible to reduce the amount of the blood to be substituted with the substitution solution (physiological saline solution or dialysate) by substituting the blood with the supplied air. Consequently, it is possible to reliably substitute the blood during returning of the blood and it is possible to suppress the supply amount of the substitution solution (In particular, in the present embodiment, it is not necessary to supply the substitution solution).

Further, the air pump 18 can supply the air to or can discharge the air from the artery side air trap chamber 5 and the vein side air trap chamber 6. Thus, the liquid surface inside the artery side air trap chamber 5 and the vein side air trap chamber 6 can be adjusted. Therefore, in addition to the function of supplying the air during returning of the blood after the treatment, it is possible to provide the function of adjusting the liquid surface in the artery side air trap chamber 5 and the vein side air trap chamber 6 before the treatment or during the treatment.

Hitherto, the present embodiment has been described. However, the present invention is not limited thereto. As long as there is provided the blood purification apparatus that includes the air circulation line which is connected to the predetermined portion of the blood circuit and can circulate the air and that can supply the air into the blood circuit via the air circulation line during returning of blood, the air pump 18 as the air supplying device may not be provided. However, if there are provided the air supplying device (air pump 18) which can supply the air into the blood circuit via the air circulation line and the control device which can control the air supplying device to supply the air into the blood circuit during returning of the blood, it is possible to more reliably and accurately supply the air into the blood circuit during returning of the blood.

In addition, for example, the control device 19 may perform the control during returning of the blood only or other substitution solutions which are different from the physiological saline solution or the dialysate may be used. Furthermore, in the present embodiment, the artery side air trap chamber 5 and the vein side air trap chamber 6 are respectively connected to the arterial blood circuit 1 and the venous blood circuit 2. However, the present embodiment may be applied to those having the air trap chamber connected to either the arterial blood circuit 1 or the venous blood circuit 2.

Furthermore, in the above-described embodiment, the air circulation line is connected to both of the artery side air trap chamber 5 and the vein side air trap chamber 6 to each of which the air can be supplied. However, instead of this manner, the air circulation line may be connected only to either the artery side air trap chamber 5 or the vein side air trap chamber 6 and the air may be supplied only to the air trap chamber thereof. In addition, in the present embodiment, the air circulation line is connected to the monitor tubes Lc and Ld, and the monitor tubes Lc and Ld are shared in use as a flow route when supplying the air. However, instead of the manner, the air circulation line may be connected to the artery side air trap chamber 5 and the vein side air trap chamber 6 without passing through the monitor tubes Lc and Ld.

The dialysis device B according to the present embodiment is connected to any separate dialysate supplying apparatus which produces the dialysate, but may also be applied to a so-called "personal dialysis apparatus" which internally has a function of producing the dialysate. In addition, in the present embodiment, any dialysis device B is applied to the hemodialysis apparatus, but may be applied to the blood purification apparatus which performs the other blood purification treatment different from the hemodialysis treatment.

### Industrial Applicability

As long as there is provided a blood purification apparatus that includes an air circulation line which is connected to a predetermined portion of a blood circuit and can circulate air and that can supply the air into the blood circuit via the air circulation line during returning of blood, it is possible to apply the blood purification apparatus to apparatuses having a different outer shape or other additional functions.

### Reference Signs List

- 1: arterial blood circuit
- 2: venous blood circuit
- 3: dialyzer (blood purification device)
- 4: blood pump
- 5: artery side air trap chamber
- 6: vein side air trap chamber
- 7, 8: liquid surface detection sensor
- 9, 10: air bubble detection sensor
- 11: containing device
- 12: air trap chamber
- 13: air bubble detection sensor
- 14: duplex pump
- 15: ultrafiltration pump
- 16, 17: pressure sensor
- 18: air pump (air supplying device)
- 19: control device

## Claims

1. A blood purification apparatus comprising:
a blood circuit that consists of an arterial blood circuit and a venous blood circuit and that can extracorporeally circulate blood of a patient from a tip of the arterial blood circuit to a tip of the venous blood circuit; and
a blood purification device that is interposed between the arterial blood circuit and the venous blood circuit in the blood circuit and that purifies the blood flowing in the blood circuit,
wherein the blood purification apparatus can perform returning of blood by substituting the blood inside the blood circuit after treatment,
wherein the blood purification apparatus further includes an air circulation line that is connected to a predetermined portion in the blood circuit and that can circulate air, and
wherein during returning of the blood, the air can be supplied into the blood circuit via the air circulation line.

2. The blood purification apparatus according to Claim 1, further comprising:
an air supplying device that can supply the air into the blood circuit via the air circulation line; and
a control device that controls the air supplying device to supply the air into the blood circuit during returning of the blood.

3. The blood purification apparatus according to Claim 1 or 2, further comprising:
an air trap chamber that is connected to the arterial blood circuit or the venous blood circuit,
wherein the air can be supplied by connecting the air circulation line to the air trap chamber.

4. The blood purification apparatus according to Claim 3, further comprising:
a liquid surface detection sensor that can detect a liquid surface of the air trap chamber,
wherein under a condition that the liquid surface sensor detects the liquid surface, the air circulation line stops supplying the air.

5. The blood purification apparatus according to Claim 3 or 4,
wherein the air supplying device can supply the air to or discharge the air from the air trap chamber and can adjust the liquid surface inside the air trap chamber.

6. The blood purification apparatus according to any one of Claims 1 to 5,
wherein an air bubble detection sensor which can detect an air bubble inside a flow route of a tip portion and a valve device which can open and close the flow route of the tip portion are arranged in the tip portion of the arterial blood circuit and the venous blood circuit, and
wherein during returning of the blood, under a condition that the air bubble detection sensor detects the air bubble, the valve device is in a closed state and the air circulation line stops supplying the air.

7. The blood purification apparatus according to any one of Claims 1 to 6, further comprising:
a containing device that contains a predetermined amount of a physiological saline solution as a substitution solution; and
a substitution solution supplying device that has a physiological saline solution supplying line which can supply the physiological saline solution inside the containing device into the blood circuit by connecting the containing device and a predetermined portion of the blood circuit to each other, and that can supply the substitution solution into the blood circuit.

8. The blood purification apparatus according to any one of Claims 1 to 6,
wherein the blood purification device can back-filtrate a dialysate as the substitution solution and supply the filtered dialysate into the blood circuit.
